Europäisches Patentamt

European Patent Office

Office européen des brevets

(19)

(11) Veröffentlichungsnummer : **0 207 088**
**B1**

(12) **EUROPÄISCHE PATENTSCHRIFT**

(45) Veröffentlichungstag der Patentschrift :
05.04.89

(51) Int. Cl.⁴ : **A 61 L 27/00**

(21) Anmeldenummer : **85905622.8**

(22) Anmeldetag : **04.11.85**

(86) Internationale Anmeldenummer :
**PCT/EP 85/00586**

(87) Internationale Veröffentlichungsnummer :
**WO/8603977 (17.07.86 Gazette 86/17)**

(54) VERFAHREN ZUR HERSTELLUNG IMPLANTIERBARER KNOCHENERSATZWERKSTOFFE.

(30) Priorität : **28.12.84 DE 3447583**

(43) Veröffentlichungstag der Anmeldung :
**07.01.87 Patentblatt 87/02**

(45) Bekanntmachung des Hinweises auf die Patenterteilung : **05.04.89 Patentblatt 89/14**

(84) Benannte Vertragsstaaten :
**AT BE CH DE FR GB IT LI LU NL SE**

(56) Entgegenhaltungen :
**EP--A-- 0 006 544**
**EP--A-- 0 023 608**
**EP--A-- 0 120 689**
**FR--A-- 2 336 913**

(73) Patentinhaber : **Battelle-Institut e.V.**
**Am Römerhof 35 Postfach 900 160**
**D-6000 Frankfurt/Main 90 (DE)**

(72) Erfinder : **HEIDE, Helmut**
**Am Hohenstein 14**
**D-6233 Kelkheim 2 (DE)**
Erfinder : **ETZKORN, Heinz-Werner**
**Schumannstrasse 12**
**D-6392 Neu-Anspach (DE)**
Erfinder : **POESCHEL, Eva**
**Sodener Weg 51**
**D-6232 Bad Soden (DE)**
Erfinder : **STEININGER, Helmut**
**Schumannstrasse 16**
**D-6392 Neu-Anspach (DE)**

(74) Vertreter : **Sartorius, Peter, Dipl.-Ing.**
**Battelle-Institut e.V. Abteilung Patente Am Römerhof**
**35**
**D-6000 Frankfurt am Main 90 (DE)**

## Beschreibung

Die Erfindung betrifft ein Verfahren zur Herstellung implantierbarer Knochenersatzwerkstoffe, bei dem auf einen metallischen Kern eine bioaktive, poröse Schicht aus Hydroxylapatit aufgebracht und durch isostatisches Heißpressen verdichtet wird.

Ein derartiges Verfahren beschreibt die EP-A1-6 544. Verglichen mit anderen bekannten Verfahren, bei denen auch ein metallischer Kern mit einer bioaktiven Schicht auf Kalziumphosphat-Basis beschichtet wird, zeichnet sich dieses bekannte Verfahren durch eine gute Verbindung zwischen dem Kern und der Beschichtung aus, die durch das isostatische Heißpressen bewirkt wird. Beim isostatischen Heißpressen können sich aber Probleme insofern ergeben, als der hierbei durch die Zersetzung des Kalziumphosphats entstehende $H_2O$ Zersetzungsdruck die Verbindung beeinträchtigen kann.

Die EP-A1-23 608 beschreibt einen Knochen-Implantatkörper, der ebenfalls aus einem mechanisch stabilen und mit Knochengewebe verträglichen Trägerwerkstoff besteht, in den bioreaktive Kalziumphosphatkeramik eingelagert ist. Zur Herstellung dieses Implantatkörpers wird als Trägerwerkstoff ein Sintermetall verwendet, das schadlos und ohne Bildung von Zwischenreaktionsprodukten mit Kalziumphosphatkeramik unter hohem Druck versintert werden kann. Die Oberflächenschicht aus reinem Kalziumphosphat wird hierbei durch einen oder mehrere weitere Druck-Sintervorgänge auf den Implantatkörper aufgepreßt. Die Praxis hat gezeigt, daß dieses vorbeschriebene Verfahren nicht allen Anforderungen genügt, insbesondere weil es nicht ausreichend die unterschiedlichen thermischen Ausdehnungskoeffizienten der Sintermaterialien berücksichtigt, die ebenfalls zu einer Lockerung bzw. Zerstörung des Verbundes führen können. Auch besteht die Gefahr, daß Metallionen aus dem Untergrund der Beschichtung in das Knochenlager gelangen können.

Ausgehend von einem Verfahren der eingangs genannten Art liegt der Erfindung die Aufgabe zugrunde, dieses so zu führen, daß mit dem Verfahren gut haftende bioaktive Schichten ohne eine nachteilige Veränderung der chemischen Natur und somit des biologischen Verhaltens hergestellt werden können.

Zur Lösung dieser Aufgabe ist das erfindungsgemäße Verfahren dadurch gekennzeichnet, daß der Druck und die Temperatur so eingestellt werden, daß eine Wasserabspaltung aus dem Hydroxylapatit vermieden wird und daß anschließend die Hydroxylapatitschicht durch Ionenbeschuß partiell entwässert wird.

Der erfindungsgemäß hergestellte Werkstoff weist in seiner Masse eine verminderte Bioaktivität auf, wird aber in seiner Grenzfläche bis zu einer Tiefe von maximal 1 μm bioaktiv gemacht. Er ist demzufolge nur in einem dünnen definierten Oberflächenbereich resorbierbar. Erfindungsgemäß wird die chemische und mechanische Stabilität der gewebeverträglichen Metalle genutzt und diese sehr wesentlichen Eigenschaften werden mit der für die Knochenneubildung verantwortlichen Bioaktivität des Schichtmaterials kombiniert. Das an sich nicht resorbierbare Hydroxylapatit, das im natürlichen Knochen vorkommt, wird durch einen energiereichen Ionenbeschuß entwässert und damit insbesondere in das resorbierbare Trikalziumphosphat überführt. Diese Umwandlung des Hydroxylapatits wird insbesondere im Oberflächenbereich vorgenommen. Die Bildung der resorbierbaren Kalziumphosphat-Phasen erfolgt dadurch ohne Sinterungsprozeß.

Als metallischer Kern wird insbesondere Titan verwendet. Auch andere, gewebeverträglichen Metalle sind geeignet. Auf einen solchen massiven Metallkern wird mittels eines Schlickers eine poröse Schicht aus Hydroxylapatit aufgebracht. Der beschichtete Metallkern wird dann bei Drücken von mehr als 100 MPa und Temperaturen von größer als 400 °C einer HIP-Behandlung (Hot Isostatic Pressing) unterzogen. Dabei wird die Beschichtung porenfrei verdichtet und fest mit der metallischen Unterlage verbunden. Weiter wird der $H_2O$-Zersetzungsdruck des Hydroxylapatits durch den hohen Innendruck der HIP-Anlage kompensiert, so daß sich das Hydroxylapatit nicht zersetzt. Im Anschluß daran wird die gereinigte Oberfläche des Implantats durch intensiven Ionenbeschuß bis zu einer Tiefe von etwa 0,1 bis 0,5 μm entwässert, wobei eine dem Tricalziumphosphat ähnliche Gitterstruktur mit einer hohen Bioaktivität gebildet wird, welche als Starterschicht für die Osteogenese dient.

Es ist vorteilhaft, die Oberfläche des metallischen Kerns vor der Beschichtung durch an sich bekannte chemische und/oder physikalische Methoden zu reinigen. Hierfür wird z. B. Ätzen, Sputtern, Plasmaspritzen und/oder Ionenimplantation vorgeschlagen. Die Haftfestigkeit der kalciumphosphatischen Schicht mit der mit der metallischen Unterlage wird weiterhin erhöht, wenn vor dem isostatischen Heißpressen eine kalte isostatische Preßbehandlung vorgenommen wird. Hierfür wird vorzugsweise ein Schlicker verwendet und eine elastische Hülle, z. B. aus Gummi. Das isostatische Heißpressen kann in einem Container aus Glas durchgeführt werden. Zur Verhinderung einer Reaktion zwischen dem Containermaterial und der Schicht wird ein Barrierematerial eingesetzt. Hierfür sind alle inerte Substanzen geeignet, insbesondere Graphit oder Bornitrid.

## Patentansprüche

1. Verfahren zur Herstellung implantierbarer Knochenersatzwerkstoffe, bei dem auf einen metallischen Kern eine bioaktive, poröse Schicht aus Hydroxylapatit aufgebracht und durch isostatisches Heißpressen verdichtet wird, dadurch gekennzeichnet, daß der Druck und die Temperatur so eingestellt werden, daß eine Wasserabspaltung aus dem Hydroxylapatit vermieden wird und daß anschließend die Hydroxylapatitschicht durch lonenbeschuß partiell entwässert wird.

2. Verfahren nach Anspruch 1, dadurch gekennzeichnet, daß das isostatische Heißpressen bei Drücken von mehr als 100 MPa und Temperaturen von mehr als 400 °C durchgeführt wird.

3. Verfahren nach Anspruch 1 oder 2, dadurch gekennzeichnet, daß die Hydroxylapatit-Schicht bis zu einer Tiefe von 0,1 bis 1,0 μm, vorzugsweise 0,5 μm entwässert wird.

4. Verfahren nach einem der Ansprüche 1 bis 3, dadurch gekennzeichnet, daß die Oberfläche des metallischen Kerns vor dem Aufbringen der Hydroxylapatit-Schicht durch chemische und/oder physikalische Methoden, vorzugsweise durch Ätzen, Sputtern, Plasmaspritzen und/oder Ionenimplantation vorbehandelt wird.

5. Verfahren nach einem der Ansprüche 1 bis 4, dadurch gekennzeichnet, daß vor dem isostatischen Heißpressen die poröse Hydroxylapatit-Schicht einem kalten isostatischen Pressen unterzogen wird.

6. Verfahren nach Anspruch 5, dadurch gekennzeichnet, daß das kalte isostatische Pressen durch einen Schlicker und unter Verwendung einer elastischen Hülle, vorzugsweise aus Gummi, durchgeführt wird.

7. Verfahren nach einem der Ansprüche 1 bis 6, dadurch gekennzeichnet, daß das isostatische Heißpressen in einem Container aus Glas durchgeführt wird und daß zur Verhinderung von Reaktionen mit der Hydroxylapatit-Schicht ein Reaktionsträges Barrierematerial, vorzugsweise Graphit oder Bornitrid, verwendet wird.

8. Verfahren nach einem der Ansprüche 1 bis 7, dadurch gekennzeichnet, daß die verdichtete Hydroxylapatit-Schicht durch naßchemische und/oder physikalische Methoden, vorzugsweise durch Ätzen und/oder Sputtern gereinigt wird.

### Claims

1. Method of producing implantable bone replacement materials, according to which a bioactive porous layer of hydroxylapatite is applied to a metallic core and compacted by hot isostatic pressing, comprising ; pressure and temperature being adjusted such that dehydration of the hydroxylapatite is avoided, and that the hydroxylapatite layer is subsequently partially dehydrated by ion bombardment.

2. Method as claimed in claim 1 wherein hot isostatic pressing is carried out at pressures above 100 MPa and temperatures above 400 °C.

3. Method as claimed in claim 1 or claim 2 wherein the hydroxylapatite layer is dehydrated down to a depth of 0.1 to 1.0 μm, preferably 0.5 μm.

4. Method as claimed in any of the claims 1 to 3 wherein, prior to application of the hydroxylapatite layer, the surface of the metallic core is pretreated by chemical and/or physical methods, preferably by etching, sputtering, plasma spraying and/or ion implantation.

5. Method as claimed in any of the claims 1 to 4 wherein, prior to hot isostatic pressing, the porous hydroxylapatite layer is subjected to cold isostatic pressing.

6. Method as claimed in claim 5 wherein cold isostatic pressing is carried out using a slip and an elastic container which is preferably made of rubber.

7. Method as claimed in any of the claims 1 to 6 wherein hot isostatic pressing is carried out in a glass container and wherein, in order to prevent reactions with the hydroxylapatite layer, an inert barrier material, preferably graphite or boron nitride, is used.

8. Method as claimed in any of the claims 1 to 7 wherein the compacted hydroxylapatite coating is cleaned by wet-chemical and/or physical methods, preferably by etching and/or sputtering.

### Revendications

1. Procédé de fabrication de matériaux de remplacement des os, implantables, dans lequel une couche poreuse, bioactive à base d'hydroxylapatite est appliquée sur un noyau métallique et compressée par pressage isostatique à chaud, caractérisé en ce que la pression et la température sont ajustées de telle sorte qu'une séparation d'eau à partir de l'hydroxylapatite soit évitée et qu'ensuite la couche d'hydroxylapatite soit partiellement déshydratée par bombardement ionique.

2. Procédé selon la revendication 1, caractérisé en ce que le pressage isostatique à chaud est effectué à des pressions de plus de 100 MPa et à des températures de plus de 400 °C.

3. Procédé selon la revendication 1 ou 2, caractérisé en ce que la couche d'hydroxylapatite est déshydratée jusqu'à une profondeur de 0,1 à 1,0 μm, de préférence 0,5 μm.

4. Procédé selon l'une des revendications 1 à 3, caractérisé en ce que la surface du noyau métallique est prétraitée avant l'application de la couche d'hydroxylapatite par des méthodes chimiques et/ou physiques, de préférence par décapage, crépitement, pulvérisation de plasma et/ou implantation d'ions.

5. Procédé selon l'une des revendications 1 à 4, caractérisé en ce que la couche poreuse d'hydroxylapatite est soumise à un pressage isostatique à froid avant le pressage isostatique à chaud.

6. Procédé selon la revendication 5, caractérisé en ce que le pressage isostatique à froid est effectué au moyen d'un applicateur de boue et en utilisant une enveloppe élastique, de préférence à base de caoutchouc.

7. Procédé selon l'une des revendications 1 à 6, caractérisé en ce que le pressage isostatique à chaud est effectué dans un container en verre et que pour empêcher des réactions avec la couche d'hydroxylapatite, un matériau faisant barrière qui supporte la réaction — de préférence du graphite ou du nitrure de bore — est utilisé.

8. Procédé selon l'une des revendications 1 à 7, caractérisé en ce que la couche compressée d'hydroxylapatite est purifiée par des méthodes de la chimie par voie humide et/ou physique, de préférence par décapage, et/ou par crépitement.